(19) Europäisches Patentamt
European Patent Office
Office européen des brevets

(11) Veröffentlichungsnummer: **0 140 270**
**A2**

(12) **EUROPÄISCHE PATENTANMELDUNG**

(21) Anmeldenummer: **84112429.0**

(22) Anmeldetag: **16.10.84**

(51) Int. Cl.⁴: **C 07 C 69/606**
C 07 C 69/65, C 07 C 67/10

(30) Priorität: **29.10.83 DE 3339350**

(43) Veröffentlichungstag der Anmeldung:
**08.05.85 Patentblatt 85/19**

(84) Benannte Vertragsstaaten:
**AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Lantzsch, Reinhard, Dr.**
**Am Buschhäuschen 51**
**D-5600 Wuppertal 1(DE)**

(72) Erfinder: **Hänssler, Gerd, Dr.**
**Heymannstrasse 40**
**D-5090 Leverkusen 1(DE)**

(72) Erfinder: **Brandes, Wilhelm, Dr.**
**Eichendorffstrasse 3**
**D-5653 Leichlingen 1(DE)**

(72) Erfinder: **Rosslenbroich, Hans-Jürgen, Dr.**
**Rembrandtstrasse 20**
**D-5019 Monheim(DE)**

(54) **4-Pentinsäure-Derivate.**

(57) 4-Pentinsäure-Derivate der allgemeinen Formel (I)

$$Z-C\equiv C-CR^1R^2-CR^3R^4-\overset{\overset{O}{\|}}{C}-X \qquad (I)$$

in welcher

$Z, R^1, R^2, R^3, R^4$ und X die in der Beschreibung angegebene Bedeutung haben

sowie ihre Verwendung als Schädlingsbekämpfungsmittel, vor allem ihre Verwendung als Fungizide und Bakterizide.

Die 4-Pentinsäure-Derivate können nach mehreren Verfahren hergestellt werden, z.B. wenn man geeignete 3-Alkinylhalogenide mit geeigneten Malonester-Derivaten umsetzt.

EP 0 140 270 A2

Croydon Printing Company Ltd

BAYER AKTIENGESELLSCHAFT 5090 Leverkusen, Bayerwerk

Konzernverwaltung RP
Patentabteilung Bas/AB
Ib

# 4-Pentinsäure-Derivate

Die Erfindung betrifft 4-Pentinsäure-Derivate, mehrere Verfahren zu ihrer Herstellung und ihre Verwendung als Schädlingsbekämpfungsmittel.

Es ist bereits bekannt, daß bestimmte Metallsalze der Dithiocarbamidsäuren, wie z.B. Zinkethylen-1,2-bis-dithiocarbamat, gute fungizide Eigenschaften aufweisen.

Die Wirkung dieser Verbindungen ist jedoch unter bestimmten Umständen, insbesondere bei niedrigen Aufwandmengen und -konzentrationen, nicht immer ganz befriedigend.

Es wurden die neuen 4-Pentinsäure-Derivate der allgemeinen Formel (I) gefunden,

$$Z-C{\equiv}C-CR^1R^2-CR^3R^4-\overset{O}{\overset{\|}{C}}-X \qquad (I)$$

in welcher

Z für Wasserstoff, Halogen, Alkyl, Alkenyl, Cycloalkenyl oder für gegebenenfalls substituiertes Aryl oder Benzyl steht,

$R^1$ und $R^2$ gleich oder verschieden sind und für Alkyl stehen,

$R^3$ für Wasserstoff, Chlor oder Brom steht,

$R^4$ für Wasserstoff oder eine -CO-Y-Gruppierung steht, in welcher

Y für Hydroxy, Alkoxy, für gegebenenfalls substituiertes Benzyloxy oder für den Rest $-NR^5R^6$ steht, in welchem

$R^5$ und $R^6$ gleich oder verschieden sind und für Wasserstoff, Alkyl, Alkenyl, Alkinyl, Benzyl oder gegebenenfalls substituiertes Aryl stehen und

X für Hydroxy, Alkoxy, für gegebenenfalls substituiertes Benzyloxy oder für den Rest $-NR^7R^8$ steht, in welchem

$R^7$ und $R^8$ gleich oder verschieden sind und für Wasserstoff, Alkyl, Alkenyl, Alkinyl, Benzyl oder gegebenenfalls substituiertes Aryl stehen,

wobei wenn

Z für Wasserstoff steht,

$R^4$ für Wasserstoff steht und

X für Hydroxy, Methoxy oder Ethoxy steht,

oder

Z für Wasserstoff steht und

X und Y beide gleichzeitig für Hydroxy oder Ethoxy stehen,

in diesen Fällen

$R^3$ für Chlor oder Brom steht.

Weiterhin wurde gefunden, daß die 4-Pentinsäure-Derivate der allgemeinen Formel (Ia),

$$Z-C\equiv C-CR^1R^2-CR^3R^4-\overset{O}{\overset{\|}{C}}-X \qquad (Ia)$$

in welcher

Z für Wasserstoff, Halogen, Alkyl, Alkenyl, Cycloalkenyl oder für gegebenenfalls substituiertes Aryl oder Benzyl steht,

$R^1$ und $R^2$ gleich oder verschieden sind und für Alkyl stehen,

$R^3$ für Wasserstoff, Chlor oder Brom steht,

$R^4$ für Wasserstoff oder eine -CO-Y-Gruppierung steht, in welcher

Y für Hydroxy, Alkoxy, für gegebenenfalls substituiertes Benzyloxy oder für den Rest $-NR^5R^6$ steht,

in welchem

$R^5$ und $R^6$ gleich oder verschieden sind und für Wasserstoff, Alkyl, Alkenyl, Alkinyl, Benzyl oder gegebenenfalls substituiertes Aryl stehen und

X für Hydroxy, Alkoxy, für gegebenenfalls substituiertes Benzyloxy oder für den Rest $-NR^7R^8$ steht, in welchem

$R^7$ und $R^8$ gleich oder verschieden sind und für Wasserstoff, Alkyl, Alkenyl, Alkinyl, Benzyl oder gegebenenfalls substituiertes Aryl stehen,

als Schädlingsbekämpfungsmittel verwendet werden können.

Die Verbindungen der Formel (I) bzw. (Ia) können als geometrische und/oder optische Isomere oder Isomerengemische unterschiedlicher Zusammensetzung anfallen. Sowohl die reinen Isomeren als auch die Isomerengemische werden erfindungsgemäß beansprucht.

Außerdem wurde gefunden, daß die neuen 4-Pentinsäure-Derivate der allgemeinen Formel (I) erhalten werden, wenn man

(a) 3-Alkinylhalogenide der Formel (II),

$$Z^1-C\equiv C-CR^1R^2-Hal \qquad (II)$$

in welcher

$Z^1$ für Wasserstoff, Alkyl, Alkenyl, Cycloalkenyl oder für gegebenenfalls substituiertes Aryl oder Benzyl steht,

$R^1$ und $R^2$ die oben angegebenen Bedeutungen haben und

Hal für Chlor oder Brom steht,

mit Malonester-Derivaten der Formel (III),

$$\begin{array}{l} \quad\;\; CO-Y^1 \\ \quad\;\; | \\ Me-CH \\ \quad\;\; | \\ \quad\;\; CO-X^1 \end{array} \qquad (III)$$

in welcher

$X^1$ und $Y^1$ für $C_1$-$C_4$-Alkoxy stehen,

Me für ein Aequivalent eines Alkali- oder Erdalkalimetallatoms, wie insbesondere Lithium, Natrium, Kalium oder Magnesium steht,

gegebenenfalls in Gegenwart eines Verdünnungsmittels und gegebenenfalls in Gegenwart von Katalysatoren umsetzt;

oder gegebenenfalls

(b) die nach dem Verfahren (a) erhaltenen 4-Pentinsäure-Derivate der Formel (Ib),

$$\begin{array}{l} \qquad\qquad\qquad\quad\; CO-Y^1 \\ \qquad\qquad\qquad\quad\; | \\ Z^1-C\equiv C-CR^1R^2-CH \\ \qquad\qquad\qquad\quad\; | \\ \qquad\qquad\qquad\quad\; CO-X^1 \end{array} \qquad (Ib)$$

in welcher

$Z^1$, $R^1$, $R^2$, $X^1$ und $Y^1$ die oben unter (a) angegebenen Bedeutungen haben,

in Gegenwart äquimolarer oder überschüssiger Menger einer Base und gegebenenfalls in Gegenwart eines Verdünnungsmittels verseift;

oder wenn man

(c) 4-Pentinsäurechloride der Formel (IV),

$$Z^1-C\equiv C-CR^1R^2-\underset{\displaystyle CO-R^{10}}{\overset{\displaystyle CO-R^9}{CH}} \qquad (IV)$$

in welcher

$Z^1$, $R^1$ und $R^2$ die oben unter (a) angegebenen Bedeutungen haben und

$R^9$ und $R^{10}$ gleich sind und für Chlor stehen, oder verschieden sind und für Chlor und Hydroxy oder $C_1$-$C_4$-Alkoxy stehen,

mit Verbindungen der Formel (V),

$$HQ \qquad (V)$$

in welcher

Q für Alkoxy, gegebenenfalls substituiertes Benzyloxy, für den Rest $-NR^5R^6$ oder für den Rest $-NR^7R^8$ steht, in welchen

$R^5$, $R^6$, $R^7$ und $R^8$ die oben angegebenen Bedeutungen haben,

gegebenenfalls in Gegenwart von Säureakzeptoren und gegebenenfalls in Gegenwart von Verdünnungsmitteln umsetzt;

oder wenn man

(d) 4-Pentinsäure-Derivate der Formel (Ic),

$$HC\equiv C-CR^1R^2-CHR^4-\overset{O}{\overset{\|}{C}}-X \qquad (Ic)$$

in welcher

$R^1$, $R^2$, $R^4$ und X die oben angegebenen Bedeutungen haben,

mit äquimolarem oder überschüssigem Halogen, wie Chlor, Brom oder Iod, gegebenenfalls in Gegenwart eines Säureakzeptors und gegebenenfalls in Gegenwart eines Verdünnungsmittels umsetzt.

Die bekannten Verbindungen der Formel (Ia) lassen sich nach mehreren bekannten Verfahren herstellen (vgl. dazu J.Biol.Chem. 175, 771 (1948); J.Org.Chem. 27, 3602 (1962); Helv.Chim. Acta. 51, 1672 (1968); DE-OS 26 38 453 bzw. die Herstellungsbeispiele).

Die teilweise bekannten 4-Pentinsäure-Derivate der Formel (Ia) weisen starke fungizide Eigenschaften auf. Dabei zeigen überraschenderweise die erfindungsgemäßen Verbindungen eine höhere Wirkung als die aus dem Stand der Technik bekannte Verbindung Zinkethylen-1,2-bis-dithiocarbamat, welche wirkungsmäßig eine naheliegende Verbindung ist.

Die erfindungsgemäßen Verbindungen sind durch die Formel (I) allgemein definiert. Bevorzugt werden die Verbindungen der Formel (I), in denen

Z für Wasserstoff, Halogen, wie Chlor, Brom oder Iod, Alkyl mit 1 bis 6 Kohlenstoffatomen, Alkenyl oder Cycloalkenyl mit jeweils 3 bis 6 Kohlenstoffatomen, für Naphthyl sowie für gegebenenfalls einfach oder mehrfach, gleich oder verschieden substituiertes Phenyl oder Benzyl steht, wobei als Substituenten vorzugsweise genannt seien : Halogen, Alkyl mit 1 bis 4 Kohlenstoffatomen, Alkoxy und Alkylthio mit jeweils 1 oder 2 Kohlenstoffatomen, Nitro, Halogenalkyl und Halogenalkoxy sowie Halogenalkylthio mit jeweils 1 oder 2 Kohlenstoffatomen und 1 bis 5 gleichen oder verschiedenen Halogenatomen, wie vorzugsweise Fluor und Chloratomen;

$R^1$ und $R^2$ gleich oder verschieden sind und für Alkyl mit 1 bis 6 Kohlenstoffatomen stehen;

$R^3$ für Wasserstoff, Chlor oder Brom steht;

$R^4$ für Wasserstoff oder eine CO-Y-Gruppierung steht, in welcher Y für Hydroxy, geradkettiges oder verzweigtes Alkoxy mit 1 bis 6 Kohlenstoffatomen, für einfach oder mehrfach, gleich oder verschieden substituiertes Benzyloxy steht, wobei als Substituenten des Phenylringes die bei Z bereits genannten Phenylsubstituenten infrage kommen; sowie für den Rest $-NR^5R^6$ steht, in welchem

$R^5$ und $R^6$ gleich oder verschieden sind und für Wasserstoff, geradkettiges oder verzweigtes Alkyl mit 1

bis 6 Kohlenstoffatomen, Alkenyl oder Alkinyl mit jeweils 2 bis 6 Kohlenstoffatomen,Benzyl sowie für gegebenenfalls einfach oder mehrfach, gleich oder verschieden substituiertes Phenyl stehen, wobei als Phenylsubstituenten die bei Z bereits genannten Phenylsubstituenten infrage kommen; und

X für Hydroxy, geradkettiges oder verzweigtes Alkoxy mit 1 bis 6 Kohlenstoffatomen, für gegebenenfalls einfach oder mehrfach, gleich oder verschieden substituiertes Benzyloxy, wobei als Substituenten des Phenylringes die bei Z bereits genannten Phenylsubstituenten infrage kommen, sowie für den Rest $-NR^7R^8$ steht, in welchem

$R^7$ und $R^8$ gleich oder verschieden sind und für Wasserstoff, geradkettiges oder verzweigtes Alkyl mit 1 bis 6 Kohlenstoffatomen, Alkenyl und Alkinyl mit jeweils 2 bis 6 Kohlenstoffatomen, Benzyl sowie für gegebenenfalls einfach oder mehrfach, gleich oder verschieden substituiertes Phenyl stehen, wobei als Phenylsubstituenten die bei Z bereits genannten Phenylsubstituenten infrage kommen;

wobei wenn

Z für Wasserstoff steht,
$R^4$ für Wasserstoff steht und
X für Hydroxy, Methoxy oder Ethoxy steht,

oder

Z für Wasserstoff steht und
X und Y beide gleichzeitig für Hydroxy oder Ethoxy stehen,

in diesen Fällen

$R^3$ für Chlor oder Brom steht.

Besonders bevorzugt sind diejenigen Verbindungen der Formel (I), in denen

Z für Wasserstoff, Chlor, Brom, Iod, Methyl, Ethyl, n-Propyl, i-Propyl, n-Butyl, i-Butyl, sec.-Butyl, tert.-Butyl, Allyl, Cyclopentenyl, Cyclohexenyl oder für gegebenenfalls einfach bis dreifach, gleich oder verschieden substituiertes Phenyl oder Benzyl steht, wobei als Substituenten der Ringe genannt seien: Fluor, Chlor, Methyl, Ethyl, Methoxy, Ethoxy, Methylthio, Trifluormethyl, Trifluormethoxy und/oder Trifluormethylthio;

$R^1$ und $R^2$ jeweils für Methyl, Ethyl, n-Propyl oder n-Butyl stehen;

$R^3$ für Wasserstoff, Chlor oder Brom steht;

$R^4$ für Wasserstoff oder eine CO-Y-Gruppierung steht, in welcher

Y für Hydroxy, Methoxy, Ethoxy, n-Propoxy, i-Propoxy, n-Butoxy, i-Butoxy oder für gegebenenfalls einfach bis dreifach, gleich oder verschieden substituiertes Benzyloxy, wobei als Substituenten des Phenylringes die bei Z bereits genannten Phenylsubstituenten infrage kommen; sowie für den Rest $-NR^5R^6$ steht, in welchem

$R^5$ und $R^6$ gleich oder verschieden sind und für Wasserstoff, Methyl, Ethyl, n-Propyl, i-Propyl, n-Butyl, i-Butyl, sec.-Butyl, tert.-Butyl, Allyl, Propargyl,

Benzyl sowie für gegebenenfalls einfach oder mehrfach, gleich oder verschieden substituiertes Phenyl stehen, wobei als Phenylsubstituenten die bei Z bereits genannten Phenylsubstituenten infrage kommen, und

X für Hydroxy, Methoxy, Ethoxy, n-Propoxy, i-Propoxy, n-Butoxy, i-Butoxy, für gegebenenfalls einfach bis dreifach, gleich oder verschieden substituiertes Benzyloxy steht, wobei als Substituenten des Phenylringes die bei Z bereits genannten Phenylsubstituenten infrage kommen; sowie für den Rest $-NR^7R^8$ steht, in welcher

$R^7$ und $R^8$ gleich oder verschieden sind und für Wasserstoff, Methyl, Ethyl, n-Propyl, i-Propyl, n-Butyl, i-Butyl, sec.-Butyl, tert.-Butyl, Allyl, Propargyl, Benzyl sowie für gegebenenfalls einfach bis dreifach, gleich oder verschieden substituiertes Phenyl stehen, wobei als Phenylsubstituenten die bei Z bereits genannten Phenylsubstituenten infrage kommen;

wobei wenn

Z für Wasserstoff steht,
$R^4$ für Wasserstoff steht und
X für Hydroxy, Methoxy oder Ethoxy steht,

oder

Z für Wasserstoff steht und
X und Y beide gleichzeitig für Hydroxy oder Ethoxy stehen,

in diesen Fällen

$R^3$ für Chlor oder Brom steht.

Im einzelnen seien außer den bei den Herstellungsbeispielen genannten Verbindungen die folgenden Verbindungen der allgemeinen Formel (I) genannt :

$$Z-C\equiv C-CR^1R^2CR^3R^4-\overset{O}{\overset{\|}{C}}-X \qquad (I)$$

Tabelle 1 :

| Z | $R^1$ | $R^2$ | $R^3$ | $R^4$ | X |
|---|---|---|---|---|---|
| I | $CH_3$ | $CH_3$ | H | $-COOC_2H_5$ | OH |
| I | $CH_3$ | $CH_3$ | H | $-COOH$ | OH |
| I | $CH_3$ | $CH_3$ | H | $-CONH_2$ | $NH_2$ |
| I | $CH_3$ | $CH_3$ | H | $-CONHC_2H_5$ | $-NHC_2H_5$ |
| 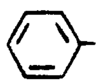 | $CH_3$ | $CH_3$ | H | $-COOC_2H_5$ | $-OC_2H_5$ |
| 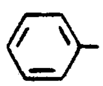 | $CH_3$ | $CH_3$ | H | $-COOH$ | $-OH$ |
| 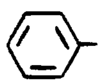 | $CH_3$ | $CH_3$ | H | $-COOC_2H_5$ | $-OH$ |
| tert-$C_4H_9$ | $CH_3$ | $CH_3$ | H | $-COOC_2H_5$ | $-OC_2H_5$ |
| tert-$C_4H_9$ | $CH_3$ | $CH_3$ | H | $-COOH$ | $-OH$ |
| tert-$C_4H_9$ | $CH_3$ | $CH_3$ | H | $-COOC_2H_5$ | OH |
| 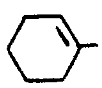 | $CH_3$ | $CH_3$ | H | $-COOC_2H_5$ | $-OC_2H_5$ |
| 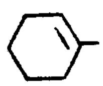 | $CH_3$ | $CH_3$ | H | $-COOH$ | $-OH$ |
| 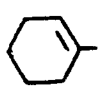 | $CH_3$ | $CH_3$ | H | $-COOH$ | $-OC_2H_5$ |

Tabelle 1 - Fortsetzung :

| Z | $R^1$ | $R^2$ | $R^3$ | $R^4$ | X |
|---|---|---|---|---|---|
| I | $CH_3$ | $CH_3$ | H | H | $-OH$ |
| I | $CH_3$ | $CH_3$ | H | H | $-OCH_3$ |
| I | $CH_3$ | $CH_3$ | H | H | $-NHC_2H_5$ |
| H | $CH_3$ | $CH_3$ | H | $-COOCH_3$ | $-OH$ |
| I | $CH_3$ | $CH_3$ | H | $-COOCH_3$ | $-OH$ |
| H | $CH_3$ | $CH_3$ | H | $-COOC_4H_9$-tert. | $-OH$ |
| I | $CH_3$ | $CH_3$ | H | $-COOC_4H_9$-tert. | $-OH$ |
| H | $CH_3$ | $CH_3$ | H | $-COOC_2H_5$ | $-NH_2$ |
| I | $CH_3$ | $CH_3$ | H | $-COOC_2H_5$ | $-NH_2$ |
| H | $CH_3$ | $CH_3$ | H | $-COOC_2H_5$ | $-NHCH_3$ |
| I | $CH_3$ | $CH_3$ | H | $-COOC_2H_5$ | $-NHCH_3$ |
| H | $CH_3$ | $CH_3$ | H | $-COOC_2H_5$ | $-NHC_2H_5$ |
| I | $CH_3$ | $CH_3$ | H | $-COOC_2H_5$ | $-NHC_2H_5$ |
| H | $CH_3$ | $CH_3$ | H | $-COOC_2H_5$ | $-N(CH_3)_2$ |
| I | $CH_3$ | $CH_3$ | H | $-COOC_2H_5$ | $-N(CH_3)_2$ |

Verwendet man beispielsweise das Natriumsalz des Malonsäuredimethylesters und 3-Chlor-3-methyl-1-butin als Ausgangssstoffe, so kann der Verlauf des erfindungsgemäßen Verfahrens (a) durch das folgende Formelschema wiedergegeben werden :

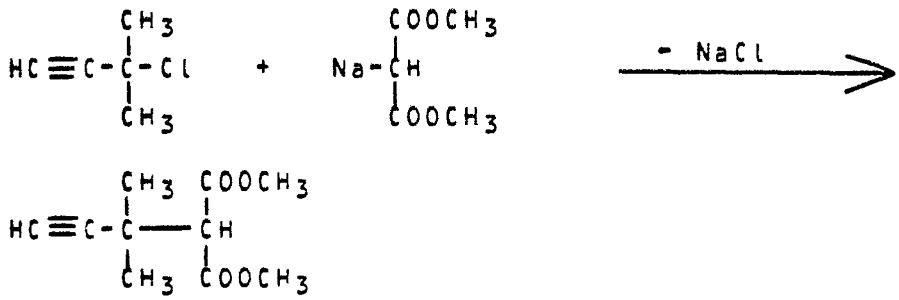


Verwendet man beispielsweise die nach Verfahren (a) hergestellte Verbindung 3,3-Dimethyl-2-methoxycarbonylpent-4-insäuremethylester und Kaliumhydroxid als Ausgangsstoffe, so kann der Verlauf des erfindungsgemäßen Verfahrens (b) durch das folgende Formelschema wiedergegeben werden :

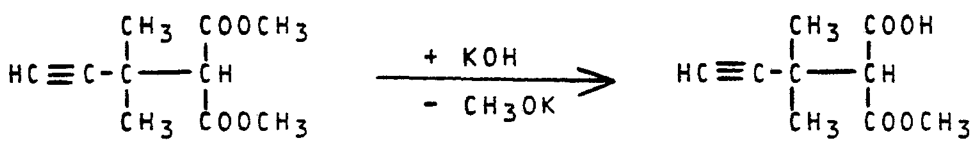


Verwendet man beispielsweise 3,3-Dimethyl-2-methoxycarbonyl-4-pentinsäurechlorid und Ammoniak als Ausgangsstoffe, so kann der Verlauf des erfindungsgemäßen Verfahrens (c) durch das folgende Formelschema wiedergegeben werden :

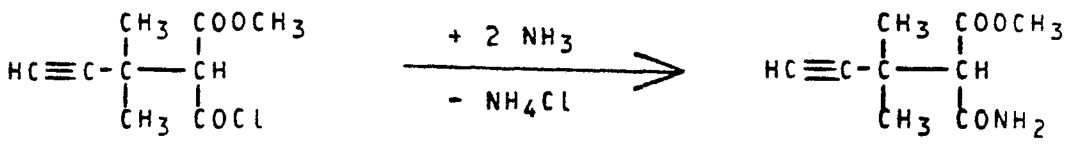


Verwendet man beispielsweise 3,3-Dimethyl-pent-4-in-säure-methylester und Brom als Ausgangsstoffe, so kann der Ablauf des erfindungsgemäßen Verfahrens (d) durch das folgende Formelschema wiedergegeben werden :

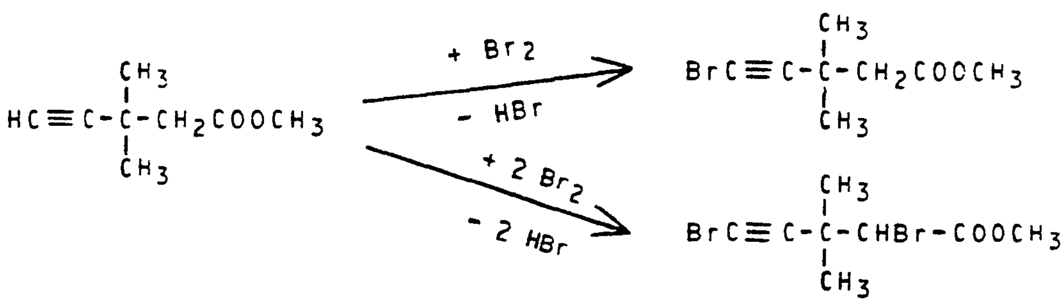


Die für die Durchführung des erfindungsgemäßen Verfahrens (a) als Ausgangsstoffe zu verwendenden 3-Alkinylhalogenide sind durch die Formel (II) allgemein definiert. In dieser Formel (II) haben $R^1$ und $R^2$ die Bedeutungen, die bereits im Zusammenhang mit der Beschreibung der erfindungsgemäßen Stoffe der Formel (I) für diese Substituenten genannt wurden. $Z^1$ steht vorzugsweise für Wasserstoff, Alkyl mit 1 bis 6 Kohlenstoffatomen, wie insbesondere Methyl, Ethyl, n-Propyl, i-Propyl, n-Butyl, i-Butyl, sec.-Butyl oder tert.-Butyl, Alkenyl und Cycloalkenyl mit 3 bis 6 Kohlenstoffatomen, wie

insbesondere Allyl, Cyclopentenyl oder Cyclohexenyl, sowie für gegebenenfalls einfach bis dreifach, gleich oder verschieden substituiertes Phenyl oder Benzyl, wobei als Substituenten des Phenylringes die Substituenten infrage kommen, die bereits im Zusammenhang mit der Beschreibung der erfindungsgemäßen Stoffe der Formel (I) vorzugsweise für diese Substituenten genannt wurden. Hal steht bevorzugt für Chlor oder Brom.

Die 3-Alkinylhalogenide sind bekannt und oder können nach an sich bekannten Verfahren hergestellt werden (vgl.dazu J.Am.Chem.Soc. 79, 2142 (1957); J.Biol.Chem. 175, 771 (1948)).

Die außerdem für die Durchführung des erfindungsgemäßen Verfahrens (a) als Ausgangsstoffe zu verwendenden Malonester-Derivate sind durch die Formel (III) allgemein definiert. In dieser Formel stehen $X^1$ und $Y^1$ für $C_1$-$C_4$-Alkoxy. Me steht vorzugsweise für ein Aequivalent eines Alkalimetalls, wie inbesondere Lithium, Natrium oder Kalium.

Die Malonester-Derivate der Formel (III) sind allgemein bekannte Verbindungen der organischen Chemie.

Die bei der Durchführung des erfindungsgemäßen Verfahrens (b) als Ausgangsstoffe zu verwendenden 4-Pentinsäure-Derivate der Formel (Ib) sind teilweise bekannt (vgl. J.Biol.Chem. 175, 771 (1948) und J.Org.Chem. 27, 3602 (1962)) und teilweise erfindungsgemäße Verbindungen.

Die bei der Durchführung des erfindungsgemäßen Verfahrens (c) als Ausgangsstoffe zu verwendenden 4-Pentinsäurechloride sind durch die Formel (IV) allgemein definiert. In dieser Formel haben $R^1$, $R^2$ und $Z^1$ vorzugsweise die Bedeutungen, die bereits im Zusammenhang mit der Beschreibung des Verfahrens (a) genannt wurden. $R^9$ und $R^{10}$ sind in dieser Formel vorzugsweise gleich und stehen für Chlor, oder sind verschieden und stehen für Chlor und Hydroxy oder $C_1$-$C_4$-Alkoxy.

Die 4-Pentinsäurechloride der Formel (IV) sind neu. Die Herstellung erfolgt nach an sich bekannten Verfahren durch Umsetzung von 4-Pentinsäure-Derivaten der Formel (Id),

$$Z^1-C\equiv C-CR^1R^2-\underset{\displaystyle CO-X^2}{\overset{\displaystyle CO-Y^2}{CH}} \qquad \text{(Id)}$$

in welcher

$Z^1$, $R^1$ und $R^2$ die unter (a) angegebenen Bedeutungen haben und

$X^2$ und $Y^2$ gleich sind und für Hydroxy stehen, oder verschieden sind und für Hydroxy und $C_1$-$C_4$-Alkoxy stehen,

mit Halogenierungsmitteln, wie z.B. Thionylchlorid, gegebenenfalls in Gegenwart von Katalysatoren, wie z.B. Dimethylformamid und gegebenenfalls in Gegenwart von Verdünnungsmitteln, wie z.B. Tetrachlorkohlenstoff, im allgemeinen bei Temperaturen zwischen 0°C und 100°C, vorzugsweise zwischen 20°C und 80°C.

Die 4-Pentinsäure-Derivate der Formel (Id) sind teilweise bekannt (vgl. J.Org.Chem. 27, 3602 (1962)). Die neuen 4-Pentinsäure-Derivate der Formel (Id) lassen sich nach dem erfindungsgemäßen Verfahren (b) herstellen.

Die außerdem für die Durchführung des erfindungsgemäßen Verfahrens (c) als Ausgangsstoffe zu verwendenden Verbindungen sind durch die Formel (V) definiert. In dieser Formel steht Q vorzugsweise für Alkoxy mit 1 bis 4 Kohlenstoffatomen, wie insbesondere Methoxy, Ethoxy, n-Propoxy, i-Propoxy, n-Butoxy, i-Butoxy, sec.-Butoxy, tert.-Butoxy; gegebenenfalls substituiertes Benzyloxy, wobei als Substituenten des Phenylringes vorzugsweise die Substituenten infrage kommen, die bereits im Zusammenhang mit der Beschreibung der erfindungsgemäßen Stoffe der Formel (I) vorzugsweise für diese Substituenten genannt wurden; für den Rest $-NR^5R^6$ sowie für den Rest $-NR^7R^8$, in welchen $R^5$, $R^6$, $R^7$ und $R^8$ die Bedeutungen haben, die bereits im Zusammenhang mit der Beschreibung der erfindungsgemäßen Verbindungen der Formel (I) für $R^5$, $R^6$, $R^7$ und $R^8$ genannt wurden.

Die Verbindungen der Formel (V) sind allgemein bekannte Verbindungen der organischen Chemie.

Die bei der Durchführung des erfindungsgemäßen Verfahrens (d) als Ausgangsstoffe zu verwendenden 4-Pentinsäure-Derivate sind durch die Formel (Ic) allgemein definiert. In dieser Formel haben $R^1$, $R^2$, $R^4$ und X die Bedeutungen, die bereits im Zusammenhang mit der Beschreibung der erfindungsgemäßen Stoffe der Formel (I) für diese Substituenten genannt wurden.

Die 4-Pentinsäure-Derivate der Formel (Ic) sind teilweise bekannt und können nach an sich bekannten Verfahren hergestellt werden (vgl. Helv.Chim.Acta 51, 1672 (1968); DE-OS 26 38 453; J.Org.Chem. 27, 2602 (1962) und J.Biol. Chem. 175, 771 (1948)).

Das erfindungsgemäße Verfahren (a) wird in Gegenwart von organischen Lösungsmitteln durchgeführt. Als organische Lösungsmittel kommen vorzugsweise Alkohole infrage. Um eine Umesterung zu vermeiden, wird vorzugsweise im gleichen Alkohol gearbeitet, mit der die Malonsäure verestert ist.

Das erfindungsgemäße Verfahren (a) kann gegebenenfalls in Gegenwart von Katalysatoren durchgeführt werden. Als Katalysatoren kommen vorzugsweise Kupfer oder Kupfer(I)-Salze, wie z.B. Kupfer(I)chlorid, infrage.

Die Reaktionstemperaturen können bei der Durchführung des erfindungsgemäßen Verfahrens (a) in einem größeren Bereich variiert werden. Im allgemeinen arbeitet man bei Temperaturen zwischen 20°C und 120°C, vorzugsweise zwischen 40°C und 100°C.

Bei der Durchführung des erfindungsgemäßen Verfahrens (a) werden die Ausgangsstoffe der Formel (II) und (III) gewöhnlich annähernd in äquimolaren Mengen eingesetzt. Ein Ueberschuß des einen oder anderen Reaktionspartners bringt keine wesentlichen Vorteile. Die Isolierung der Endprodukte erfolgt in allgemein üblicher Weise.

Als Verdünnungsmittel kommen für das erfindungsgemäße Verfahren (b) vorzugsweise Alkohole und Wasser bzw. Gemische beider infrage.

Als Basen kommen für das erfindungsgemäße Verfahren (b) alle üblicherweise verwendbaren anorganischen Basen in Betracht. Hierzu gehören vorzugsweise Alkalicarbonate, wie z.B. Natrium- und Kaliumcarbonat; sowie Alkalihydroxide, wie z.B. Natriumhydroxid.

Bei der Durchführung des erfindungsgemäßen Verfahrens (b) werden für die Herstellung der Verbindungen der Formel (Id), in welcher $X^2$ und $Y^2$ für Hydroxy stehen, auf 1 Mol der Verbindung der Formel (Ib) mindestens 2 Aequivalente Base oder aber ein noch größerer Ueberschuß eingesetzt.

Die Temperaturen können in einem größeren Bereich variiert werden. Im allgemeinen arbeitet man zwischen 20°C und 120°C, vorzugsweise zwischen 20°C und 100°C.

Bei der Durchführung des erfindungsgemäßen Verfahrens (b) werden für die Herstellung der Verbindungen der Formel (Id), in welcher $X^2$ und $Y^2$ verschieden sind, auf 1 Mol der Verbindung der Formel (Ib) 1 bis 1,2, vorzugsweise 1 Aequivalent Base eingesetzt.

Im allgemeinen arbeitet man bei Reaktionstemperaturen zwischen -20°C und +50°C, vorzugsweise zwischen 0°C und 25°C.

Die Isolierung der Endprodukte nach Verfahren (b) erfolgt in allgemein üblicher Weise.

Das erfindungsgemäße Verfahren (c) kann ohne Verdünnungsmittel oder in Gegenwart von Wasser oder organischen Lösungsmitteln durchgeführt werden. Hierzu gehören Ether, wie Tetrahydrofuran und Dioxan; Kohlenwasserstoffe, wie Benzin, Hexan, Petrolether, Cyclohexan, Toluol und Benzol; sowie chlorierte Kohlenwasserstoffe, wie Chloroform, Me-

0140270

thylenchlorid, Tetrachlorkohlenstoff und Chlorbenzol.

Als Basen kommen für das erfindungsgemäße Verfahren (c) alle üblicherweise verwendbaren anorganischen und organischen Basen in Betracht. Hierzu gehören vorzugsweise Alkalicarbonate, wie z.B. Natrium- und Kaliumcarbonat; Alkalihydroxide, wie z.B. Natriumhydroxid; Alkalialkoholate, wie z.B. Natrium- und Kalium-methylat und -ethylat; Alkalihydride, wie z.B. Natriumhydrid; sowie niedere tertiäre Alkylamine, Cycloalkylamine und Aralkylamine, wie insbesondere Triethylamin.

Gegebenenfalls kann auch in überschüssigem Amin der Formel $HNR^5R^6$ oder $HNR^7R^8$ gearbeitet werden.

Die Reaktionstemperaturen können bei der Durchführung des erfindungsgemäßen Verfahrens (c) in einem größeren Bereich variiert werden. Im allgemeinen arbeitet man bei Temperaturen zwischen -20°C und +50°C, vorzugsweise zwischen 0°C und 30°C.

Bei der Durchführung des erfindungsgemäßen Verfahrens (c) setzt man auf 1 Mol 4-Pentinsäurechlorid der Formel (IV) vorzugsweise 1 bis 2 Mol der Verbindung der Formel (V), sowie 1 bzw. 2 Mol Base ein.

Die Isolierung erfolgt in allgemein üblicher Weise.

Für das erfindungsgemäße Verfahren (d) kommen als Verdünnungsmittel vorzugsweise protische Lösungsmittel infrage. Hierzu gehören vorzugsweise Alkohole, wie Methanol, Ethanol, Propanol, Isopropanol und Glykolmonomethylether. Gegebenenfalls kann auch in Mischungen derselben mit anderen Verdünnungsmitteln, beispielsweise Wasser oder mit organischen Aminen, wie Pyridin, Chinolin oder Picolinen, gearbeitet werden.

Das erfindungsgemäße Verfahren (d) kann auch in einem Zweiphasensystem, wie beispielsweise wässrige Natron- oder Kalilauge/Toluol oder Methylenchlorid, unter Zusatz von 0,01 bis 1 Mol eines Phasen-Transfer-Katalysators, wie beispielsweise Ammonium- oder Phosphoniumverbindungen, durchgeführt werden, wobei in der organischen Phase oder an der Grenzfläche die Alkoholate entstehen und mit der in der organischen Phase befindlichen Halogeniden umgesetzt werden.

Die erfindungsgemäße Umsetzung (d) wird in Gegenwart von basischen Verbindungen ausgeführt. Als basische Verbindungen können Metallhydroxide, beispielsweise Hydroxide von Alkali-, Erdalkali- und Metallen der III. Hauptgruppe des Periodensystems, nach Mendelejew eingesetzt werden. Bevorzugt werden Alkali- und Erdalkalihydroxide wie Natrium-, Kalium-, Calcium- und Bariumhydroxid eingesetzt. Besonders bevorzugt werden Natrium- und Kaliumhydroxid eingesetzt. Die basischen Verbindungen werden bevorzugt in einem Verdünnungsmittel gelöst eingesetzt. Besonders bevorzugt sind wässrige Lösungen.

Die Reaktionstemperaturen können bei dem erfindungsgemäßen Verfahren (d) in einem größeren Bereich variiert werden. Im allgemeinen arbeitet man bei Temperaturen zwischen -20°C und +80°C, vorzugsweise zwischen +10°C und +50°C.

Das erfindungsgemäße Verfahren (d) kann kontinuierlich und diskontinuierlich ausgeführt werden.

Bei der Durchführung des erfindungsgemäßen Verfahrens (c) können Halogen und gegebenenfalls eine Base gleichzeitig zur vorgelegten 4-Pentinsäure-Derivat der Formel (Ic) gegeben werden.

Es kann auch eine Mischung von Halogen mit einer Base zum vorgelegten 4-Pentinsäure-Derivat der Formel (Ic) gegeben werden.

Weiterhin ist es möglich, Halogen und gegebenenfalls eine basische Verbindung vorzulegen und 4-Pentinsäure-Derivat der Formel (Ic) zuzugeben.

Weiterhin kann es vorteilhaft sein, Halogen und 4-Pentinsäure-Derivat der Formel (Ic) vorzulegen und dann gegebenenfalls eine basische Verbindung zuzugeben. Nachdem die Umsetzung beendet ist, wird auf übliche Art und Weise aufgearbeitet.

Die neuen Verbindungen fallen zum Teil in Form von Oelen an, die sich zum Teil nicht unzersetzt destillieren lassen, jedoch durch sogenanntes 'Andestillieren', d.h. durch längeres Erhitzen unter vermindertem Druck auf mäßig erhöhte Temperaturen von den letzten flüchtigen Anteilen befreit und auf diese Weise gereinigt werden. Zu ihrer Charakterisierung dienen der Brechungsindex oder die $^{1}H$-NMR-Spektren.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes und Deuteromycetes.

Beispielhaft aber nicht begrenzend seien einige Erreger von pilzlichen Erregern, die unter die oben aufgezählten Oberbegriffen fallen, genannt:

Botrytis-Arten, wie beispielsweise Botrytis cinerea;
Plasmopara-Arten, wie beispielsweise Plasmopara viticola,
Uromyces-Arten, wie beispielsweise Uromyces appendiculatus;
Sphaerotheca-Arten, wie beispielsweise Sphaerotheca fuliginea;
Venturia-Arten, wie beispielsweise Venturia inaequalis;
Podosphaera-Arten, wie beispielsweise Podosphaera leucotricha;
Phytophthora-Arten, wie beispielsweise Phytophthora infestans;
Erysiphe-Arten, wie beispielsweise Erysiphe graminis;
Puccinia-Arten, wie beispielsweise Puccinia recondita;
Fusarium-Arten, wie beispielsweise Fusarium culmorum;
Ustilago-Arten, wie beispielsweise Ustilago nuda oder avenae,

Septoria-Arten, wie beispielsweise Septoria nodorum;
Tilletia-Arten, wie beispielsweise Tilletia caries;

Pyricularia-Arten, wie beispielsweise Pyricularia oryzae;
Pellicularia-Arten, wie beispielsweise Pellicularia sasakii;

Pyrenophora-Arten, wie beispielsweise Pyrenophora teres (Konidienform: Drechslera, Syn: Helminthosporium);
Leptosphaeria-Arten, wie beispielsweise Leptosphaeria nodorum;
Cochliobolus-Arten, wie beispielsweise Cochliobolus sativus;
(Konidienform: Drechslera, Syn: Helminthosporium)
und
Cercospora-Arten, wie beispielsweise Cercospora canescens.

Die gute Pflanzenverträglichkeit der Wirkstoffe der Formel (Ia) in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel können die Wirkstoffe z.B. mit guten Erfolg zur Bekämpfung von Gemüsekrankheiten, wie beispielsweise gegen den Erreger der Tomatenbraunfäule (Phytophthora infestans) oder von Reiskrankheiten, wie beispielsweise Pyricularia oryzae und Pellicularia sasakii eingesetzt werden. Außerdem können die Wirkstoffe bei kombinierter Anwendung mit Insektiziden, wie z.B. Carbamaten und Pyrethroiden, als Synergisten eingesetzt werden.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und /oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsaure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-ester, Polyoxyethylen-Fettalkohol-ether, z.B. Alkylaryl-polyglykol-ether, Alkylsulfonate, Alkylsulfate, Aryl-sulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Oele sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Herstellungsbeispiele

Beispiel 1

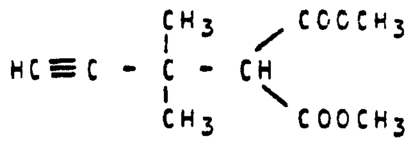

(Verfahren a)

5,06 g (0,22 Mol) Natrium werden in 100 ml trockenem Methanol gelöst und 29,04g (0,22 Mol) Malonsäuredimethylester bei 20°C zugetropft. Nach 30 Minuten Nachrühren wird innerhalb von 15 Minuten 20,5 g (0,2 Mol) 3-Chlor-3-methyl-1-butin bei 60°C zugetropft. Nach 1-stündigem Nachrühren bei 60°C wird abgekühlt, etwas eingeengt, auf Wasser gegeben und neutral gestellt. Nach dreimaligem Extrahieren mit Methylenchlorid wird fraktioniert destilliert.

Man erhält 17,2g (43,4 % der Theorie) 3,3-Dimethyl-2-methoxycarbonyl-pent-4-insäuremethylester vom Siedepunkt Kp: 56-57°C/0,1 mbar.

Beispiel 2

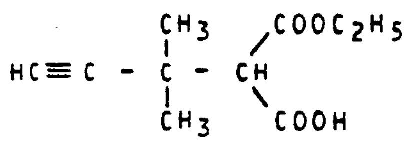

(Verfahren b)

54,9g (0,98 Mol) Kaliumhydroxid werden in 330 ml Ethanol gelöst. Dann tropft man 184,7g (0,817 Mol) 3,3-Dimethyl-2-ethoxycarbonyl-pent-4-insäureethylester (vergleiche Beispiel 20) bei 20°C zu und rührt 24 Stunden nach.

Nach Ansäuern mit verdünnter Salzsäure wird mit Methylenchlorid mehrmals extrahiert. Die vereinigten Extrakte werden getrocknet und das Lösungsmittel abdestilliert.

Der Rückstand wird im Hochvakuum bei 40°C 'andestilliert'.

Man erhält 153,1g (96 % der Theorie) 3,3-Dimethyl-2-ethoxycarbonyl-pent-4-insäure. Die Struktur wird durch $^1$H-NMR-Spektren sichergestellt.

Beispiel 3

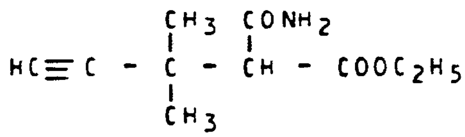

(Verfahren c)

Zu überschüssiger flüssigem Ammoniak tropft man 3,031 g (0,014 Mol) 3,3-Dimethyl-2-ethoxycarbonyl-pent-4-insäurechlorid und läßt bei 20°C 12 Stunden stehen. Man versetzt dann mit Wasser und extrahiert mit Methylenchlorid. Man erhält 2,4g (87 % der Theorie) 3,3-Dimethyl-2-ethoxycarbonyl-pent-4-insäureamid vom Schmelzpunkt 80°C.

Beispiel 4

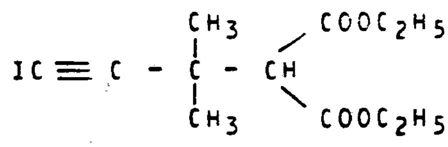

(Verfahren d)

33,9g (0,15 Mol) 3,3-Dimethyl-2-ethoxycarbonyl-pent-4-insäureethylester (vergleiche Beispiel 20) und 38,1 g (0,15 Mol) Iod werden in 160 ml Ethanol vorgelegt. Dann wird eine Lösung aus 18,48g (0,33 Mol) Kaliumhydroxid in 37 ml Wasser bei 5°C unter Kühlung zugetropft. Man rührt noch 3 Stunden bei 5°C nach, verdünnt mit Wasser und extrahiert dreimal mit Methylenchlorid. Die vereinigten Extrakte werden getrocknet, eingeengt und im Hochvakuum destilliert.
Man erhält 31,8g (60,2 % der Theorie) 3,3-Dimethyl-5-iod-ethoxycarbonyl-pent-4-insäureethylester vom Siedepunkt Kp: 98-105°C/0,3 mbar.

Beispiel 5

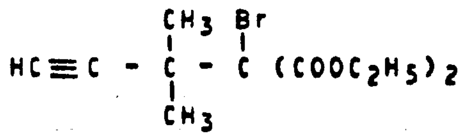

(Verfahren d)

Zu einer Lösung aus 22,6g (0,1 Mol) 3,3-Dimethyl-2-ethoxycarbonyl-pent-4-insäureethylester (vergleiche Beispiel 20) in 100 ml Chloroform tropft man 4,7 ml Brom. Man rührt bei 20°C nach, bis die Bromwasserstoffentwicklung aufgehört hat, engt ein und destilliert fraktioniert.

Man erhält 16,3g (53,4 % der Theorie) 2-Brom-3,3-dimethyl-2-ethoxycarbonyl-pent-4-insäureethylester vom Siedepunkt Kp:88-90°C/0,05 mbar.

Beispiel 6

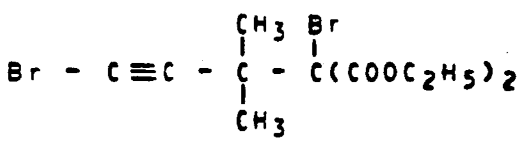

(Verfahren d)

Zu einer Lösung aus 84g (1,5 Mol) Kaliumhydroxid in 375 ml Wasser wird bei 0°C 11,7 ml Brom zugetropft. Dann tropft man bei 20°C 22,6g (0,1 Mol) 3,3-Dimethyl-2-ethoxycarbonyl-pent-4-insäureethylester (vergleiche Beispiel 20) gelöst in 50 ml Dioxan zu. Nach 30 Minuten Nachrühren wird ausgeethert, die Etherphasen vereinigt und mit Wasser neutral gewaschen, getrocknet und eingeengt. Das so erhaltene Rohprodukt wird fraktioniert destilliert.

Man erhält 15,6g (44,1 % der Theorie) 2,5-Dibrom-3,3-dimethyl-2-ethoxycarbonyl-pent-4-insäureethylester vom Siedepunkt Kp:105-110°C/0,05 mbar.

## Herstellung der Ausgangsprodukte der Formel (IV)

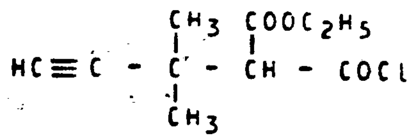

(IV-1)

14,65 g (0,074 Mol) 3,3-Dimethyl-2-ethoxycarbonyl-pent-4-insäure (vgl. Beispiel 2) werden in 150 ml Tetrachlorkohlenstoff gelöst und zwei Tropfen Dimethylformamid zugegeben. Dann tropft man bei 40-45°C 11,9 g (0,1 Mol) Thionylchlorid zu und erhitzt so lange zum Sieden, bis keine Gasentwicklung mehr zu beobachten ist. Nach dem Einengen hinterbleiben 16 g (100 % der Theorie) 3,3-Dimethyl-2-ethoxycarbonyl-pent-4-insäurechlorid.

Analog Beispiel (IV-1) wird aus 3,3-Dimethyl-2-hydroxycarbonyl-pent-4-insäure (vergleiche Beispiel 22) folgende Verbindung hergestellt:

Bsp.Nr: (IV-2) $HC \equiv C - C(CH_3)_2-CH(COCl)_2$

Analog und entsprechend den Herstellungsbeispielen 1 bis 6 bzw. Verfahren (a) bis (d) werden die nachfolgenden Verbindungen der allgemeinen Formel (I) erhalten:

$$Z - C \equiv C - CR^1R^2-CR^3R^4-\overset{O}{\overset{\|}{C}}-X \quad (I)$$

| Bsp. Nr. | Z | $R^1$ | $R^2$ | $R^3$ | $R^4$ | X | Physikal. Konstante |
|---|---|---|---|---|---|---|---|
| 7 | H | $CH_3$ | $CH_3$ | H | $-COOC_3H_7$-n | $-OC_3H_7$-n | Kp:79-80°C/ 0,2 mbar |
| 8 | H | $CH_3$ | $CH_3$ | H | $-COOC_3H_7$-i | $-OC_3H_7$-i | Kp:60°C/0,05mbar |
| 9 | H | $CH_3$ | $CH_3$ | H | $-COOC_4H_9$-n | $-OC_4H_9$-n | Kp:81-82°C/ 0,05 mbar |
| 10 | H | $CH_3$ | $CH_3$ | H | $-COOC_2H_5$ | $-N(CH_3)_2$ | Oel |
| 11 | H | $CH_3$ | $CH_3$ | H | $-COOC_2H_5$ | -NH- 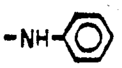 | Fp:95-96°C |
| 12 | H | $CH_3$ | $CH_3$ | H | $-COOC_2H_5$ | 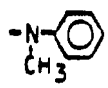 | Oel |
| 13 | H | $CH_3$ | $CH_3$ | H | $-COOC_2H_5$ | $-NH-C_3H_7$-i | Fp:70-71°C |
| 14 | H | $CH_3$ | $-C_3H_7$-n | H | $-COOC_2H_5$ | $-OC_2H_5$ | Kp:78-80°C / 0,1 mbar |
| 15 | H | $CH_3$ | $CH_3$ | H | $-CO-NH_2$ | $-NH_2$ | Fp:226-230°C |
| 16 | H | $CH_3$ | $CH_3$ | H | $-CON(CH_3)_2$ | $-N(CH_3)_2$ | Fp:142°C |
| 17 | H | $CH_3$ | $CH_3$ | H | $-CON(C_2H_5)_2$ | $-N(C_2H_5)_2$ | Fp:120°C |
| 18 | I | $CH_3$ | $-C_3H_7$-n | H | $-COOC_2H_5$ | $-OC_2H_5$ | Kp:90°C/0,05mbar |
| 19 | I | $CH_3$ | $CH_3$ | H | H | $-OC_2H_5$ | Kp:61-62°C/0,1mbar |

Die bekannten Verbindungen der Formel (Ia) werden entsprechend den nachfolgenden Herstellungsbeispielen erhalten:

Beispiel 20

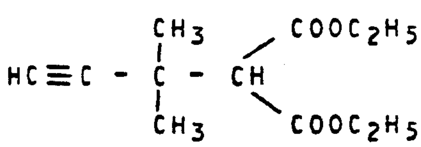

Zu 48,5g Natrium in 1000ml (790g) Ethanol werden bei 20°C 342g Malonsäurediethylester zugetropft. Anschließend werden 205g (2 Mol) 3-Chlor-3-methyl-1-butin bei 70°C innerhalb von 45 Minuten zugetropft und 1 Stunde unter Rückfluß erhitzt. Nach dem Abkühlen wird noch 2 bis 3 Stunden nachgerührt, abfiltriert, mit Ethanol nachgewaschen

und einrotiert. Der zurückbleibende Sirup wird mit eiskalter 1n Salzsäure neutral oder schwach sauer gestellt und zweimal mit insgesamt 200 ml Methylenchlorid extrahiert. Nach dem Trocknen mit Natriumsulfat wird eingeengt und anschließend fraktioniert destilliert. Man erhält 248 g (55 % der Theorie) 3,3-Dimethyl-2-ethoxycarbonyl-pent-4-insäureethylester vom Siedepunkt 72-78°C/0,08 mbar (vgl. J. Biol. Chem. 175, 771 (1948)).

Beispiel 21

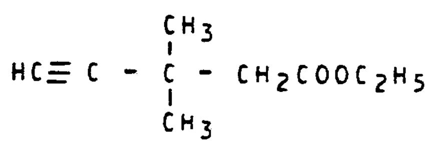

67,8g (0,3 Mol) 3,3-Dimethyl-2-ethoxycarbonyl-pent-4-insäureethylester (vergleiche Beispiel 20), 50 ml 1-Oxo-1-ethyl-phospholin, 20 g Tetraethylammoniumchlorid und 10g Wasser werden 12 Stunden auf 180°C erhitzt. Nach dem Abkühlen wird unter vermindertem Druck fraktioniert destilliert. Der Vorlauf von 12 g besteht im wesentlichen aus Ethanol. Der Hauptlauf siedet bei 60 bis 70°C/25 mbar und besteht aus 34,5g (75 % der Theorie) 3,3-Dimethyl-4-pentin-säureethylester (vergleiche DE-OS 26 38 453).

Beispiel 22

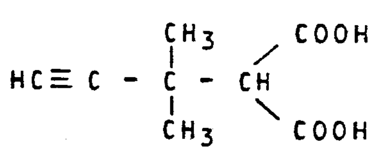

Zu 38,08g (0,68 Mol) Kaliumhydroxid in 227 ml Ethanol werden 38,42g (0,17 Mol) 3,3-Dimethyl-2-ethoxycarbonyl-pent-4-insäureethylester getropft und 12 Stunden bei 25°C gerührt. Man engt ein, stellt mit Salzsäure sauer und extrahiert mit Methylenchlorid. Die vereinigten Methylenchloridphasen werden getrocknet und das Lösungsmittel entfernt. Der Rückstand wird aus Diisopropylether/Petrolether umkristallisiert. Man erhält 25,6 g (88,6 % der Theorie) 3,3-Dimethyl-2-hydroxy-carbonyl-pent-4-insäure vom Schmelzpunkt 110 bis 113°C (vgl. J. Org. Chem. 27, 3602 (1962)).

Verwendungsbeispiele

In den nachfolgenden Verwendungsbeispielen wird die nachstehend angegebene Verbindung als Vergleichsverbindung eingesetzt:

(A) 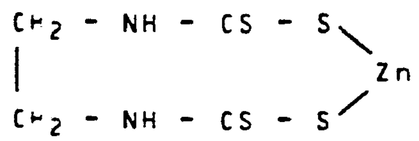

Beispiel A

Pyricularia-Test (Reis) / protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß der Herstellungsbeispiele: (2), (4), (3) und (10).

Beispiel B

Pyricularia-Test (Reis) / systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß der Herstellungsbeispiele: (1), (2), (4), (13), (14), (18), (19) und (20).

Beispiel C

Phytophthora-Test (Tomate) / kurativ

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und Emulgator und verdünnt das Konzentrat mit Wasser auf die gewünschte Konzentration.

Zur Prüfung auf kurative Wirksamkeit werden junge Pflanzen mit einer wäßrigen Sporensuspension von Phytophthora infestans inokuliert. Die Pflanzen verbleiben 7 Stunden bei 20°C und 100 % relativer Luftfeuchtigkeit in einer Inkubationskabine. Nach einer kurzen Abtrocknungszeit werden die Pflanzen mit der Wirkstoffzubereitung tropfnaß gespritzt.

Die Pflanzen werden in einer Inkubationskabine mit 100 % relativer Luftfeuchtigkeit und ca. 20°C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäß der Herstellungsbeispiele:
(3) und (10).

## Patentansprüche

1. 4-Pentinsäure-Derivate der allgemeinen Formel (I)

$$Z-C{\equiv}C-CR^1R^2-CR^3R^4-\overset{O}{\overset{\|}{C}}-X \qquad (I)$$

in welcher

Z für Wasserstoff, Halogen, Alkyl, Alkenyl, Cycloalkenyl oder für gegebenenfalls substituiertes Aryl oder Benzyl steht,

$R^1$ und $R^2$ gleich oder verschieden sind und für Alkyl stehen,

$R^3$ für Wasserstoff, Chlor oder Brom steht,

$R^4$ für Wasserstoff oder eine -CO-Y-Gruppierung steht, in welcher

Y für Hydroxy, Alkoxy, für gegebenenfalls substituiertes Benzyloxy oder für den Rest $-NR^5R^6$ steht, in welchem

$R^5$ und $R^6$ gleich oder verschieden sind und für Wasserstoff, Alkyl, Alkenyl, Alkinyl, Benzyl oder gegebenenfalls substituiertes Aryl stehen und

X für Hydroxy, Alkoxy, für gegebenenfalls substituiertes Benzyloxy oder für den Rest $-NR^7R^8$ steht, in welchem

$R^7$ und $R^8$ gleich oder verschieden sind und für Wasserstoff, Alkyl, Alkenyl, Alkinyl, Benzyl oder gegebenenfalls substituiertes Aryl stehen,

wobei wenn

Z für Wasserstoff steht,

$R^4$ für Wasserstoff steht und

X für Hydroxy, Methoxy oder Ethoxy steht,

oder

Z für Wasserstoff steht und

X und Y beide gleichzeitig für Hydroxy oder Ethoxy stehen,

in diesen Fällen

$R^3$ für Chlor oder Brom steht.

2. 4-Pentinsäure-Derivate gemäß Anspruch 1, wobei in der Formel (I)

Z für Wasserstoff, Halogen, Alkyl mit 1 bis 6 Kohlenstoffatomen, Alkenyl oder Cycloalkenyl mit jeweils 3 bis 6 Kohlenstoffatomen, für Naphthyl sowie für gegebenenfalls einfach oder mehrfach, gleich oder verschieden substituiertes Phenyl oder Benzyl steht, wobei als Substituenten genannt seien: Halogen, Alkyl mit 1 bis 4 Kohlenstoffatomen, Alkoxy und Alkylthio mit jeweils 1 oder 2 Kohlenstoffatomen, Nitro, Halogenalkyl und Halogenalkoxy sowie Halogenalkylthio mit jeweils 1 oder 2 Kohlenstoffatomen und 1 bis 5 gleichen oder verschiedenen Halogenatomen;

$R^1$ und $R^2$ gleich oder verschieden sind und für Alkyl mit 1 bis 6 Kohlenstoffatomen stehen;

$R^3$ für Wasserstoff, Chlor oder Brom steht;

$R^4$ für Wasserstoff oder eine CO-Y-Gruppierung steht, in welcher Y für Hydroxy, geradkettiges oder verzweigtes Alkoxy mit 1 bis 6 Kohlenstoffatomen, für einfach oder mehrfach, gleich oder verschieden substituiertes Benzyloxy, wobei als Substituenten des Phenylringes die bei Z bereits genannten Phenylsubstituenten in Frage kommen; oder für den Rest $-NR^5R^6$ steht, in welchem

$R^5$ und $R^6$ gleich oder verschieden sind und für Wasserstoff, geradkettiges oder verzweigtes Alkyl mit 1 bis 6 Kohlenstoffatomen, Alkenyl oder Alkinyl mit jeweils 2 bis 6 Kohlenstoffatomen, Benzyl oder für gegebenenfalls einfach oder mehrfach, gleich oder verschieden substituiertes Phenyl stehen, wobei als Phenylsubstituenten die bei Z bereits genannten Phenylsubstituenten infrage kommen; und

X für Hydroxy, geradkettiges oder verzweigtes Alkoxy mit 1 bis 6 Kohlenstoffatomen, für gegebenenfalls einfach oder mehrfach, gleich oder verschieden substituiertes Benzyloxy, wobei als Substituenten des Phenylringes die bei Z bereits genannten Phenylsubstituenten in Frage kommen, oder für den Rest $-NR^7R^8$ steht, in welchem

$R^7$ und $R^8$ gleich oder verschieden sind und für Wasserstoff, geradkettiges oder verzweigtes Alkyl mit 1 bis 6 Kohlenstoffatomen, Alkenyl oder Alkinyl mit jeweils 2 bis 6 Kohlenstoffatomen, Benzyl oder für gegebenenfalls einfach oder mehrfach, gleich oder verschieden substituiertes Phenyl stehen, wobei als Phenylsubstituenten die bei Z bereits genannten Phenylsubstituenten in Frage kommen;

wobei wenn

Z für Wasserstoff steht,

$R^4$ für Wasserstoff steht und

X für Hydroxy, Methoxy oder Ethoxy steht,

oder

Z für Wasserstoff steht und

X und Y beide gleichzeitig für Hydroxy oder Ethoxy stehen,

in diesen Fällen

$R^3$ für Chlor oder Brom steht.

3. 4-Pentinsäure-Derivate gemäß Anspruch 1, wobei in der Formel (I)

Z für Wasserstoff, Chlor, Brom, Iod, Methyl, Ethyl, n-Propyl, i-Propyl, n-Butyl, i-Butyl, sec.-Butyl, tert.-Butyl, Allyl, Cyclopentenyl, Cyclohexenyl oder für gegebenenfalls einfach bis dreifach, gleich oder verschieden substituiertes Phenyl oder Benzyl steht, wobei als Substituenten der Ringe genannt seien:

Fluor, Chlor, Methyl, Ethyl, Methoxy, Ethoxy, Methylthio, Trifluormethyl, Trifluormethoxy und/oder Trifluormethylthio;

$R^1$ und $R^2$ jeweils für Methyl, Ethyl, n-Propyl oder n-Butyl stehen;

$R^3$ für Wasserstoff, Chlor oder Brom steht;

$R^4$ für Wasserstoff oder eine CO-Y-Gruppierung steht, in welcher

Y für Hydroxy, Methoxy, Ethoxy, n-Propoxy, i-Propoxy, n-Butoxy, i-Butoxy oder für gegebenenfalls einfach bis dreifach, gleich oder verschieden substituiertes Benzyloxy steht, wobei als Substituenten des Phenylringes die bei Z bereits genannten Phenylsubstituenten in Frage kommen; oder für den Rest $-NR^5R^6$ steht, in welchem

$R^5$ und $R^6$ gleich oder verschieden sind und für Wasserstoff, Methyl, Ethyl, n-Propyl, i-Propyl, n-Butyl, i-Butyl, sec.-Butyl, tert.-Butyl, Allyl, Propargyl, Benzyl oder für gegebenenfalls einfach oder mehrfach, gleich oder verschieden substituiertes Phenyl stehen, wobei als Phenylsubstituenten die bei Z bereits genannten Phenylsubstituenten in Frage kommen, und

X für Hydroxy, Methoxy, Ethoxy, n-Propoxy, i-Propoxy, n-Butoxy, i-Butoxy, für gegebenenfalls einfach bis dreifach, gleich oder verschieden substituiertes Benzyloxy steht, wobei als Substituenten des Phenylringes die bei Z bereits genannten Phenylsubstituenten in Frage kommen; oder für den Rest $-NR^7R^8$ steht, in welchem

$R^7$ und $R^8$ gleich oder verschieden sind und für Wasserstoff, Methyl, Ethyl, n-Propyl, i-Propyl, n-Butyl, i-Butyl, sec.-Butyl, tert.-Butyl, Allyl, Propargyl, Benzyl oder für gegebenenfalls einfach bis dreifach, gleich oder verschieden substituiertes Phenyl stehen, wobei als Phenylsubstituenten die bei Z bereits genannten Phenylsubstituenten in Frage kommen;

wobei wenn

Z für Wasserstoff steht,

$R^4$ für Wasserstoff steht und

X für Hydroxy, Methoxy oder Ethoxy steht,

oder

Z für Wasserstoff steht und

X und Y beide gleichzeitig für Hydroxy oder Ethoxy stehen,

in diesen Fällen

$R^3$ für Chlor oder Brom steht.

4. Verfahren zur Herstellung von 4-Pentinsäure-Derivaten der allgemeinen Formel (I)

$$Z-C\equiv C-CR^1R^2-CR^3R^4-\overset{O}{\overset{\|}{C}}-X \qquad (I)$$

in welcher

Z für Wasserstoff, Halogen, Alkyl, Alkenyl, Cycloalkenyl oder für gegebenenfalls substituiertes Aryl oder Benzyl steht,

$R^1$ und $R^2$ gleich oder verschieden sind und für Alkyl stehen,

$R^3$ für Wasserstoff, Chlor oder Brom steht,

$R^4$ für Wasserstoff oder eine -CO-Y-Gruppierung steht, in welcher

Y für Hydroxy, Alkoxy, für gegebenenfalls substituiertes Benzyloxy oder für den Rest $-NR^5R^6$ steht, in welchem

$R^5$ und $R^6$ gleich oder verschieden sind und für Wasserstoff, Alkyl, Alkenyl, Alkinyl, Benzyl oder gegebenenfalls substituiertes Aryl stehen und

X für Hydroxy, Alkoxy, für gegebenenfalls substituiertes Benzyloxy oder für den Rest $-NR^7R^8$ steht, in welchem

$R^7$ und $R^8$ gleich oder verschieden sind und für Wasserstoff, Alkyl, Alkenyl, Alkinyl, Benzyl oder gegebenenfalls substituiertes Aryl stehen,

wobei wenn

Z für Wasserstoff steht,

$R^4$ für Wasserstoff steht und

X für Hydroxy, Methoxy oder Ethoxy steht,

oder

Z für Wasserstoff steht und

X und Y beide gleichzeitig für Hydroxy oder Ethoxy stehen,

in diesen Fällen

$R^3$ für Chlor oder Brom steht,

dadurch gekennzeichnet, daß man

(a) 3-Alkinylhalogenide der Formel (II)

$$Z^1-C{\equiv}C-CR^1R^2-Hal \qquad (II)$$

in welcher

$Z^1$ für Wasserstoff, Alkyl, Alkenyl, Cycloalkenyl oder für gegebenenfalls substituiertes Aryl oder Benzyl steht,

$R^1$ und $R^2$ die oben angegebenen Bedeutungen haben und

Hal für Chlor oder Brom steht,

mit Malonester-Derivaten der Formel (III)

$$Me-CH(CO-Y^1)(CO-X^1) \qquad (III)$$

in welcher

$X^1$ und $Y^1$ für $C_1$-$C_4$-Alkoxy stehen,

Me für ein Äquivalent eines Alkali- oder Erdalkalimetallatoms steht,

gegebenenfalls in Gegenwart eines Verdünnungsmittels und gegebenenfalls in Gegenwart von Katalysatoren umsetzt;

oder gegebenenfalls

(b) die nach dem Verfahren (a) erhaltenen 4-Pentinsäure-Derivate der Formel (Ib)

$$Z^1-C\equiv C-CR^1R^2-\underset{\underset{CO-X^1}{|}}{\overset{\overset{CO-Y^1}{|}}{CH}} \qquad (Ib)$$

in welcher

$Z^1$, $R^1$, $R^2$, $X^1$ und $Y^1$ die oben angegebenen Bedeutungen haben,

in Gegenwart äquimolarer oder überschüssiger Mengen einer Base und gegebenenfalls in Gegenwart eines Verdünnungsmittels verseift;

oder daß man

(c) 4-Pentinsäurechloride der Formel (IV)

$$Z^1-C\equiv C-CR^1R^2-\underset{\underset{CO-R^{10}}{|}}{\overset{\overset{CO-R^9}{|}}{CH}} \qquad (IV)$$

in welcher

$Z^1$, $R^1$ und $R^2$ die oben angegebenen Bedeutungen haben und

$R^9$ und $R^{10}$ gleich sind und für Chlor stehen, oder verschieden sind und für Chlor und Hydroxy oder $C_1$-$C_4$-Alkoxy stehen,

mit Verbindungen der Formel (V)

$$HQ \qquad (V)$$

in welcher

Q für Alkoxy, gegebenenfalls substituiertes Benzyloxy, für den Rest $-NR^5R^6$ oder für den Rest $-NR^7R^8$ steht, in welchen

$R^5$, $R^6$, $R^7$ und $R^8$ die oben angegebenen Bedeutungen haben,

gegebenenfalls in Gegenwart von Säureakzeptoren und gegebenenfalls in Gegenwart von Verdünnungsmitteln umsetzt;

oder daß man

(d) 4-Pentinsäure-Derivate der Formel (Ic)

$$HC{\equiv}C-CR^1R^2-CHR^4-\overset{O}{\overset{\|}{C}}-X \qquad (Ic)$$

in welcher

$R^1$,$R^2$,$R^4$ und X die oben angegebenen Bedeutungen haben,

mit äquimolarem oder überschüssigem Halogen, gegebenenfalls in Gegenwart eines Säureakzeptors und gegebenenfalls in Gegenwart eines Verdünnungsmittels umsetzt.

5. Schädlingsbekämpfungsmittel, gekennzeichnet durch einen Gehalt an mindestens einem 4-Pentinsäure-Derivat der Formel (Ia)

$$Z-C{\equiv}C-CR^1R^2-CR^3R^4-\overset{O}{\overset{\|}{C}}-X \qquad (Ia)$$

in welcher

Z für Wasserstoff, Halogen, Alkyl, Alkenyl, Cycloalkenyl oder für gegebenenfalls substituiertes Aryl oder Benzyl steht,

$R^1$ und $R^2$ gleich oder verschieden sind und für Alkyl stehen,

$R^3$ für Wasserstoff, Chlor oder Brom steht,

$R^4$ für Wasserstoff oder eine -CO-Y-Gruppierung steht, in welcher

Y für Hydroxy, Alkoxy, für gegebenenfalls substituiertes Benzyloxy oder für den Rest $-NR^5R^6$ steht, in welchem

$R^5$ und $R^6$ gleich oder verschieden sind und für Wasserstoff, Alkyl, Alkenyl, Alkinyl, Benzyl oder gegebenenfalls substituiertes Aryl stehen und

X für Hydroxy, Alkoxy, für gegebenenfalls substituiertes Benzyloxy oder für den Rest $-NR^7R^8$ steht, in welchem

$R^7$ und $R^8$ gleich oder verschieden sind und für Wasserstoff, Alkyl, Alkenyl, Alkinyl, Benzyl oder gegebenenfalls substituiertes Aryl stehen.

6. Schädlingsbekämpfungsmittel, gekennzeichnet durch einen Gehalt an mindestens einem 4-Pentinsäure-Derivat gemäß Anspruch 5, wobei in der Formel (Ia)

Z für Wasserstoff, Halogen, Alkyl mit 1 bis 6 Kohlenstoffatomen, Alkenyl oder Cycloalkenyl mit jeweils 3 bis 6 Kohlenstoffatomen, für Naphthyl oder für gegebenenfalls einfach oder mehrfach, gleich oder verschieden substituiertes Phenyl oder Benzyl steht, wobei als Substituenten genannt seien: Halogen, Alkyl mit 1 bis 4 Kohlenstoffatomen, Alkoxy und Alkylthio mit jeweils 1 oder 2 Kohlenstoffatomen, Nitro, Halogenalkyl und Halogenalkoxy oder Halogenalkylthio mit jeweils 1 oder 2 Kohlenstoffatomen und 1 bis 5 gleichen oder verschiedenen Halogenatomen;

$R^1$ und $R^2$ gleich oder verschieden sind und für Alkyl mit 1 bis 6 Kohlenstoffatomen stehen;

$R^3$ für Wasserstoff, Chlor oder Brom steht;

$R^4$ für Wasserstoff oder eine CO-Y-Gruppierung, in welcher Y für Hydroxy, geradkettiges oder verzweigtes Alkoxy mit 1 bis 6 Kohlenstoffatomen, für einfach oder mehrfach, gleich oder verschieden substituiertes Benzyloxy steht, wobei als Substituenten des Phenylringes die bei Z bereits genannten Phenylsubstituenten in Frage kommen; oder für den Rest $-NR^5R^6$ steht, in welchem

$R^5$ und $R^6$ gleich oder verschieden sind und für Wasserstoff, geradkettiges oder verzweigtes Alkyl mit 1 bis 6 Kohlenstoffatomen, Alkenyl oder Alkinyl mit jeweils 2 bis 6 Kohlenstoffatomen, Benzyl oder für gegebenenfalls einfach oder mehrfach, gleich oder verschieden substituiertes Phenyl stehen, wobei als Phenylsubstituenten die bei Z bereits genannten Phenylsubstituenten in Frage kommen; und

X für Hydroxy, geradkettiges oder verzweigtes Alkoxy mit 1 bis 6 Kohlenstoffatomen, für gegebenenfalls einfach oder mehrfach gleich oder verschieden substituiertes Benzyloxy steht, wobei als Substituenten des Phenylringes die bei Z bereits genannten Phenylsubstituenten in Frage kommen, oder für den Rest $-NR^7R^8$ steht, in welchem

$R^7$ und $R^8$ gleich oder verschieden sind und für Wasserstoff, geradkettiges oder verzweigtes Alkyl mit 1 bis 6 Kohlenstoffatomen, Alkenyl oder Alkinyl mit jeweils 2 bis 6 Kohlenstoffatomen, Benzyl oder für gegebenenfalls einfach oder mehrfach, gleich oder verschieden substituiertes Phenyl stehen, wobei als Phenylsubstituenten die bei Z bereits genannten Phenylsubstituenten in Frage kommen.

7. Schädlingsbekämpfungsmittel, gekennzeichnet durch einen Gehalt an mindestens einem 4-Pentinsäure-Derivat gemäß Anspruch 5, wobei in der Formel (Ia)

Z für Wasserstoff, Chlor, Brom, Iod, Methyl, Ethyl, n-Propyl, i-Propyl, n-Butyl, i-Butyl, sec.-Butyl, tert.-Butyl, Allyl, Cyclopentenyl, Cyclohexenyl oder für gegebenenfalls einfach bis dreifach, gleich oder verschieden substituiertes Phenyl oder Benzyl steht, wobei als Substituenten der Ringe genannt seien: Fluor, Chlor, Methyl, Ethyl, Methoxy, Ethoxy, Methylthio, Trifluormethyl, Trifluormethoxy und/oder Trifluormethylthio;

$R^1$ und $R^2$ jeweils für Methyl, Ethyl, n-Propyl und n-Butyl stehen;

$R^3$ für Wasserstoff, Chlor oder Brom steht;

$R^4$ für Wasserstoff oder eine CO-Y-Gruppierung steht, in welcher

Y für Hydroxy, Methoxy, Ethoxy, n-Propoxy, i-Propoxy, n-Butoxy, i-Butoxy oder für gegebenenfalls einfach bis dreifach, gleich oder verschieden substituiertes Benzyloxy, wobei als Substituenten des Phenylringes die bei Z bereits genannten Phenylsubstituenten in Frage kommen; sowie für den Rest $-NR^5R^6$ steht, in welchem

$R^5$ und $R^6$ gleich oder verschieden sind und für Wasserstoff, Methyl, Ethyl, n-Propyl, i-Propyl, n-Butyl, i-Butyl, sec.-Butyl, tert.-Butyl, Allyl, Propargyl, Benzyl sowie für gegebenenfalls einfach oder mehrfach, gleich oder verschieden substituiertes Phenyl stehen, wobei als Phenylsubstituenten die bei Z bereits genannten Phenylsubstituenten in Frage kommen, und

X für Hydroxy, Methoxy, Ethoxy, n-Propoxy, i-Propoxy, n-Butoxy, i-Butoxy, für gegebenenfalls einfach bis dreifach, gleich oder verschieden substituiertes Benzyloxy steht, wobei als Substituenten des Phenylringes die bei Z bereits genannten Phenylsubstituenten in Frage kommen; sowie für den Rest $-NR^7R^8$ steht, in welchem

$R^7$ und $R^8$ gleich oder verschieden sind und für Wasserstoff, Methyl, Ethyl, n-Propyl, i-Propyl, n-Butyl, i-Butyl, sec.-Butyl, tert.-Butyl, Allyl, Propargyl, Benzyl oder für gegebenenfalls einfach

bis dreifach, gleich oder verschieden substituiertes Phenyl stehen, wobei als Phenylsubstituenten die bei Z bereits genannten Phenylsubstituenten in Frage kommen.

8. Verfahren zur Bekämpfung von Schädlingen, dadurch gekennzeichnet, daß man 4-Pentinsäure-Derivate der Formel (Ia) gemäß Anspruch 5 auf Schädlinge oder ihren Lebensraum einwirken läßt.

9. Verwendung von 4-Pentinsäure-Derivaten der Formel (Ia) gemäß Anspruch 5 als Schädlingsbekämpfungsmittel.

10. Verfahren zur Herstellung von Schädlingsbekämpfungsmitteln, dadurch gekennzeichnet, daß man 4-Pentinsäure-Derivate der Formel (Ia) gemäß Anspruch 5 mit Streckmitteln und/oder oberflächenaktiven Mitteln vermischt.